# FASCICULE DE BREVET EUROPEEN

(11) **EP 4 108 417 B1**
(45) Date de publication et mention de la délivrance du brevet: **24.01.2024**
(21) Numéro de dépôt: 22180032.9
(22) Date de dépôt: 21.06.2022
(51) Int. Cl.: B29C 64/106, A61F 2/00, B29C 64/314, B29C 64/35, B29C 64/40, B29C 71/02, B33Y 10/00, B33Y 40/20, B33Y 70/00, B33Y 80/00

(54) **PROCÉDÉ DE RÉALISATION D'UN SUBSTITUT TISSULAIRE IMPRIMÉ EN 3D**
VERFAHREN ZUR HERSTELLUNG EINES 3D-GEDRUCKTEN GEWEBEERSATZES
METHOD FOR MANUFACTURING A 3D-PRINTED SUBSTITUTE TISSUE

(30) Priorité: 24.06.2021 FR 2106791
(43) Date de publication de la demande: 28.12.2022
(73) Titulaire: SEGULA ENGINEERING, 92000 Nanterre (FR); Université Claude Bernard Lyon 1, 69622 Villeurbanne (FR); Centre national de la recherche scientifique, 75016 Paris (FR)
(72) Inventeur: LEMARIE, Lucas, 69100 Villeurbanne (FR); COURTIAL, Edwin-Joffrey, 69100 Villeurbanne (FR)
(74) Mandataire: IPAZ

(56) Documents cités:
- CN-A- 111 574 816
- US-A1- 2017 361 534
- US-A1- 2019 275 746

## Description

L'invention concerne la réalisation par impression 3D d'un substitut tissulaire destiné à être implanté dans un corps humain.

Aujourd'hui, les dispositifs médicaux destinés à être implantés dans le corps humain (stents, endoprothèses, prothèse vasculaires, etc.) sont réalisés de préférence en biomatériau, pour permettre une régénération in situ de l'organe ou de la partie d'organe remplacée provisoirement.

On connait des procédés de fabrication de tels dispositifs par moulage et le matériau utilisé est par exemple de l'alcool polyvinylique (ou PVAL) ou du polyéthylène glycol. L'alcool polyvinylique peut être associé, dans certaines compositions, à du triméthaphosphate de sodium et à de la gélatine pour permettre la réalisation par moulage d'un dispositif.

US 2019/275746 A1 divulgue un procédé de réalisation d'un substitut tissulaire comportant une étape de solubilisation du matériau à imprimer, une étape d'alimentation d'un dispositif d'impression 3D avec le matériau solubilisé, une étape d'impression du matériau dans un fluide de contrainte dans une cuve, par déplacement de la tête d'impression dans la cuve de manière à former un dispositif intermédiaire, une étape de gélation du dispositif intermédiaire, une étape de retrait du dispositif intermédiaire solidifié de la cuve et une étape de rinçage du dispositif intermédiaire solidifié.

On connait également des dispositifs réalisés par impression 3D tels que ceux décrits dans le document WO 2016/090286. Le procédé met en oeuvre un dispositif d'impression 3D comprenant une tête d'impression équipé d'au moins une cartouche de délivrance d'un matériau à imprimer, ledit dispositif comportant une cuve comprenant un fluide de contrainte dans lequel ledit matériau est imprimé, ladite tête d'impression étant montée mobile en déplacement hors de ladite cuve et dans ladite cuve.

Pour obtenir le substitut tissulaire, il est nécessaire d'appliquer un traitement chimique et physique après l'impression 3D pour figer la forme du dispositif imprimé par réticulation et solidification : cela est obtenu en appliquant un rayonnement lumineux UV ou par des procédés de thermo-gélification.

L'inconvénient d'un tel procédé est qu'il met en oeuvre un traitement chimique pouvant rendre le dispositif potentiellement cytotoxique. L'invention vise à proposer un procédé qui ne nécessite aucun traitement chimique pour réaliser le substitut tissulaire.

L'invention concerne à cet effet un procédé de réalisation d'un substitut tissulaire imprimé en 3D, mettant en oeuvre un dispositif d'impression 3D comprenant une tête d'impression équipée d'au moins une cartouche de délivrance d'un matériau à imprimer, ledit dispositif comportant une cuve comprenant un fluide de contrainte dans lequel ledit matériau est imprimé, ladite tête d'impression étant montée mobile en déplacement, ledit matériau à imprimer se solidifiant sensiblement sous une première température de solidification et ledit fluide de contrainte se solidifiant sensiblement sous une seconde température de solidification.

Le procédé conforme à l'invention est remarquable en ce que ledit matériau à imprimer, délivré par la cartouche, comporte de l'alcool polyvinylique et de la gélatine, en ce que ladite seconde température de solidification du fluide de contrainte est inférieure à ladite première température de solidification dudit matériau à imprimer et en ce que ledit procédé comporte les étapes suivantes :
- solubilisation dudit matériau à imprimer, de manière à obtenir un matériau suffisamment fluide pour qu'il soit délivré par ladite cartouche,
- alimentation dudit dispositif d'impression 3D avec ledit matériau solubilisé,
- impression dudit matériau dans ledit fluide de contrainte dans la cuve, par déplacement de la tête d'impression dans ladite cuve et délivrance de ladite matière par ladite cartouche de manière à former un dispositif intermédiaire,
- solidification dudit dispositif intermédiaire en plaçant ladite cuve, pendant un premier intervalle de temps, à une température inférieure à ladite première température de solidification, ladite température étant toutefois supérieure de ladite seconde température de solidification, puis en plaçant la cuve à température ambiante pendant un second intervalle de temps, la température ambiante étant supérieure audites première et seconde températures,
- retrait dudit dispositif intermédiaire solidifié de ladite cuve,
- rinçage dudit dispositif intermédiaire solidifié, et
- séchage dudit dispositif intermédiaire pour obtenir ledit substitut tissulaire.

En choisissant un fluide de contrainte dont la température de solidification est inférieure à la température de solidification du matériau à imprimer et en utilisant les propriétés de réticulation de l'alcool polyvinylique à basse température, le procédé permet ainsi de créer un substitut tissulaire en biomatériau imprimé en 3D sans réticulation chimique par voie photonique (UV), ce qui évite de rendre le substitut tissulaire cytotoxique.

Le procédé conforme à l'invention peut également comprendre les caractéristiques suivantes, prises séparément ou en combinaison :
Avantageusement, ledit matériau à imprimer comporte sensiblement entre 10 et 25% en poids d'alcool polyvinylique (ou entre 10 et 25% en poids d'alcool polyvinylique) et sensiblement entre 0,5 et 2% en poids de gélatine (ou entre 0,5 et 2% en poids de gélatine), de préférence sensiblement 20% en poids d'alcool polyvinylique (ou 20% en poids d'alcool polyvinylique) et sensiblement 1% en poids de gélatine (ou 1% en poids de gélatine).

Dans le cadre d'un mode de réalisation avantageux, le fluide de contrainte comporte sensiblement au moins 80% en poids d'éthanol (ou au moins 80% en poids d'éthanol) et sensiblement entre 2 et 5% en poids d'un polymère synthétique d'acide acrylique réticulé (ou entre 2 et 5% en poids d'un polymère synthétique d'acide acrylique réticulé) commercialisé sous le nom de Carbopol^{®} (940), de préférence sensiblement 3,5% en poids (ou 3,5% en poids).

De préférence encore, la solubilisation dudit matériau à imprimer est réalisée à une température comprise sensiblement entre 90°C et 100 °C (ou entre 90°C et 100 °C).

En outre, ladite solidification dudit dispositif intermédiaire est réalisée à une température comprise sensiblement entre -5°C et -90°C (ou entre -5°C et - 90°C), de préférence à sensiblement -80°C (ou à -80°C). Avantageusement, ladite solidification est réalisée en plaçant ladite cuve à ladite température de solidification dans une enceinte réfrigérée pendant sensiblement 1 heure, puis en laissant la cuve à température ambiante pendant sensiblement 3 heures.

Pour permettre de simuler un vieillissement du substitut tissulaire créé, l'étape de solidification peut être répétée au moins deux fois.

De préférence, le séchage est réalisé par lyophilisation du dispositif intermédiaire durant un temps de lyophilisation tel que le substitut tissulaire obtenu, à l'issue dudit temps de lyophilisation, comprend moins de 3% d'eau en poids.

En outre, suivant un mode de réalisation qui sera décrit par la suite, le procédé comporte une ultime étape de décontamination dudit substitut tissulaire, par exemple en soumettant ledit substitut tissulaire à un rayonnement décontaminant (pour ne pas avoir à mettre en contact le dispositif avec un fluide potentiellement cytotoxique).

L'invention concerne également un substitut tissulaire obtenu par ledit procédé tel que défini ci-dessus.

D'autres avantages et caractéristiques de l'invention apparaîtront à l'examen de la description détaillée d'un mode de mise en oeuvre nullement limitatif, et des dessins annexés, sur lesquels :
[Fig. 1] est une représentation schématique d'une étape d'un procédé conforme à l'invention,
[Fig. 2] est une autre représentation schématique d'un dispositif d'impression 3D et d'une cuve mis en oeuvre dans une étape d'un procédé conforme à l'invention,
[Fig. 3] est une représentation schématique du dispositif et de la cuve qui sont illustrés en figure 2, mis en oeuvre suivant une autre étape d'un procédé conforme à l'invention,
[Fig. 4] illustre encore une autre étape d'un procédé conforme à l'invention,
[Fig. 5] illustre une ultime étape d'un procédé conforme à l'invention.

Le matériel mis en oeuvre dans le procédé conforme à l'invention est illustré sur les figures.

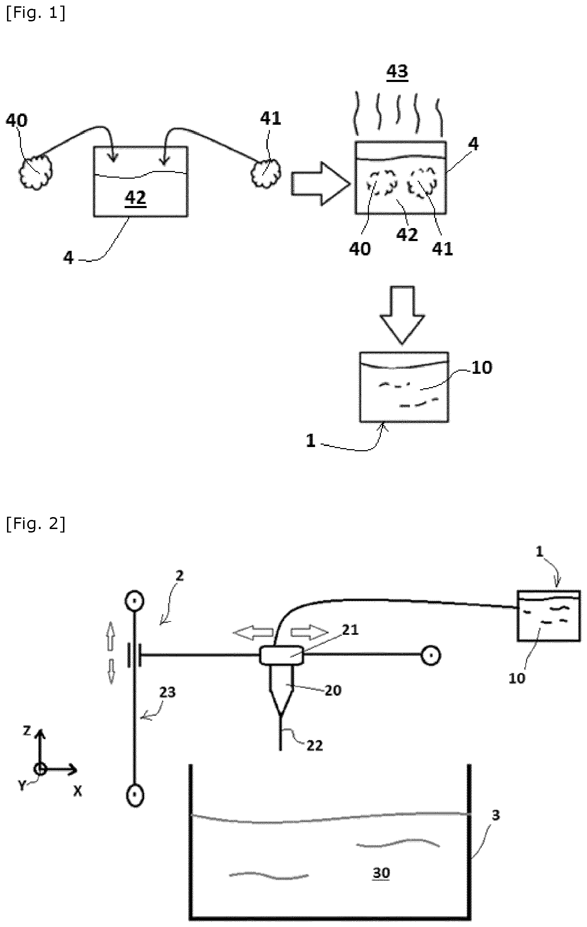

La figure 1 montre un réservoir 1 contenant un matériau à imprimer 10.

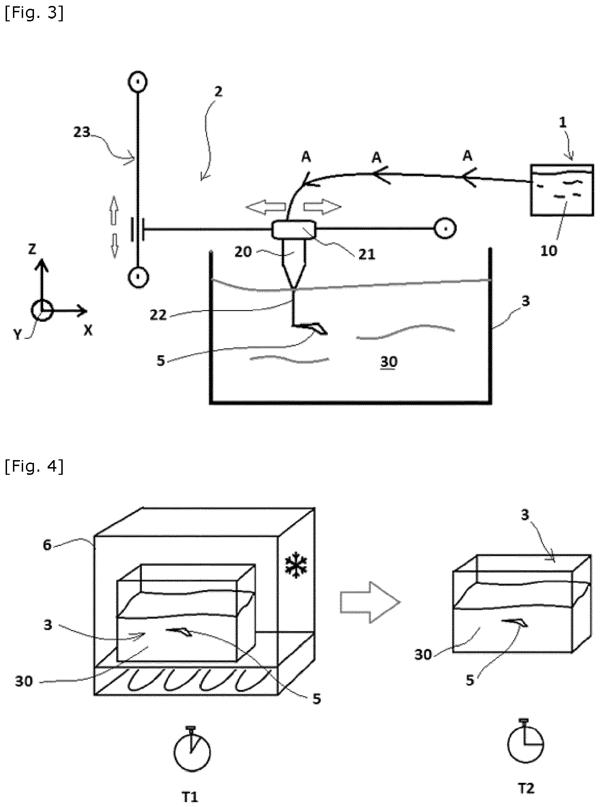

Les figures 2 et 3 illustrent un dispositif 2 d'impression 3D, comportant une tête d'impression 21 qui est équipée d'une cartouche de délivrance 20 du matériau 10 à imprimer, la tête d'impression 21 étant alimentée en matériau à imprimer 10 par le réservoir 1.

La cartouche 20 est équipée d'une aiguille 22, par laquelle le matériau 10 à imprimer est délivré.

Les figures 2 et 3 illustrent également une cuve 3, placée sous la tête d'impression 21 du dispositif 2 d'impression 3D.

La cuve 3 comporte un fluide de contrainte 30.

La tête d'impression 21 du dispositif 2 d'impression 3D est montée mobile en déplacement suivant trois directions X, Y et Z sur un châssis 23, ce qui assure le déplacement de la tête d'impression 21 hors de la cuve et dans la cuve 3, pour permettre d'imprimer en 3D un substitut tissulaire dans le fluide de contrainte 30.

Conformément à l'invention, le matériau à imprimer 10 et le fluide de contrainte 30 sont choisis de sorte que la température de solidification du matériau à imprimer 10 soit supérieure à la température de solidification du fluide de contrainte 30 : autrement dit, en abaissant la température, le matériau à imprimer 10 sera le premier qui se solidifiera parmi le matériau à imprimer 10 et le fluide de contrainte 30.

Autrement dit encore, la température de solidification du fluide de contrainte 30 est inférieure à la température de solidification du matériau à imprimer 10.

Le matériau à imprimer est, par ailleurs, un biomatériau, c'est-à-dire un matériau qui se dégrade pour permettre une régénération in situ de l'organe ou de la partie d'organe remplacée provisoirement par ce matériau.

Dans le cadre de cet exemple de mise en oeuvre d'un procédé conforme à l'invention, le matériau à imprimer comporte de l'alcool polyvinylique et de la gélatine.

Conformément à l'invention, la quantité d'alcool polyvinylique dans le matériau à imprimer 10 est comprise entre 10 et 25% en poids, ou sensiblement comprise entre 10 et 25% en poids, et la quantité de gélatine dans le matériau à imprimer 10 est comprise entre 0,5 et 2% en poids, ou sensiblement comprise entre 0,5 et 2% en poids.

La figure 1 montre (de façon schématique) la réalisation du biomatériau à imprimer 10.

Dans le cadre de cet exemple précis, le biomatériau à imprimer 10 comporte 20% en poids de polyvinyle (ou sensiblement 20% en poids), 1% en poids de gélatine (ou sensiblement 1% en poids), le troisième ingrédient étant de l'eau ultra pure (appelée encore « eau milliQ » ou « UPW ») présent à 79% en poids (ou sensiblement 79% en poids).

Dans un récipient 4, on mélange ainsi 20g d'alcool polyvinylique 40, 1g de gélatine 41 et 79g d'eau milliQ 42.

Puis, on solubilise l'ensemble pour réaliser le matériau à imprimer 10. Pour ce faire, on place le récipient à une température 43 comprise entre 90° et 100° (ou sensiblement comprise entre 90° et 100°), de préférence à 90° (ou sensiblement 90°).

L'étape de solubilisation permet d'obtenir un matériau à imprimer 10 qui soit suffisamment fluide pour être délivré par la cartouche 20 à travers l'aiguille 22.

La gélatine 41 est une gélatine porcine, de type A. De manière générale, on choisira une gélatine d'origine animale, de type médicale et destinée à un usage en laboratoire.

Dans le cadre de cet exemple, on prévoit d'ajouter un colorant au matériau imprimable : en effet, la cuve 3, le fluide de contrainte 30 et le biomatériau à imprimer 10 sont tous trois transparents. Si l'on souhaite observer l'objet qui s'imprime en 3D dans le fluide de contrainte 30, il est préférable d'ajouter un colorant au biomatériau à imprimer.

Ainsi, le dispositif imprimé en 3D est visualisable plus facilement et il est plus aisé de surveiller sa réalisation et de le saisir pour l'évacuer de la cuve 3.

Il est à noter que la température de solidification du biomatériau à imprimer (ou matériau à imprimer 10) est sensiblement de 0°C. En effet, ce sont les molécules d'eau présentes dans le biomatériau que le procédé cherche à solidifier. Aussi, dès que l'on place le biomatériau à une température inférieure à 0°C, on obtient la solidification souhaitée. Plus la température est basse sous 0 et plus la solidification se fait rapidement.

La cryo-réticulation est initiée dès la formation de cristaux par solidification de l'eau contenu dans le biomatériau. C'est ainsi que l'on obtient le substitut tissulaire.

Concernant le fluide de contrainte 30, il comporte 80% en poids d'éthanol (ou sensiblement 80% en poids d'éthanol), de l'eau pure et entre 2 et 5% en poids (ou sensiblement entre 2 et 5% en poids) d'un agent gélifiant comportant un polymère synthétique d'acide acrylique réticulé commercialisé sous le nom de CARBOPOL^{®} (Carbopol 940).

Plus précisément, la quantité de CARBOPOL^{®} est de 3,5% en poids (ou sensiblement 3,5% en poids).

Le fluide de contrainte 30 obtenu se présente alors sous la forme d'un gel. La température de solidification du fluide de contrainte est inférieure à - 80°C : elle est sensiblement de -100 °C.

La figure 3 illustre schématiquement l'impression 3D d'un dispositif intermédiaire 5 dans le fluide de contrainte 30 par le dispositif d'impression 2.

Pour ce faire, le dispositif 2 d'impression 3D est alimenté en matériau à imprimer 10 solubilisé (cette alimentation est symbolisée par les flèches A).

On commande l'impression et le dispositif 2 d'impression 3D imprime le dispositif intermédiaire dans le fluide de contrainte 30, par déplacement de la tête d'impression 21 sur le châssis 23 suivant un programme préétabli qui dépend de la forme que l'on souhaite donner au dispositif intermédiaire 5 et ses dimensions.

Pour permettre une bonne introduction de l'aiguille 22 dans le fluide de contrainte 30, il est prévu que l'aiguille présente une longueur d'au moins 5 cm.

Le fluide de contrainte 30 formant un gel maintien en position le filament imprimé : le dispositif intermédiaire imprimé est ainsi maintenu en suspension par le fluide de contrainte 30 dans la cuve 3.

Un fois le dispositif intermédiaire 5 réalisé, et conformément à l'invention, il est solidifié pour figer sa forme.

Pour ce faire, comme illustré en figure 4 de façon schématique, la cuve 3 est placée dans une enceinte réfrigérée 6, comme un congélateur, où la température est au moins de -5°C.

Il devra être noté que la température de l'enceinte réfrigérée doit toutefois rester supérieure à la température de solidification du fluide de contrainte qui, lui, doit rester liquide ou visqueux pour permettre de retirer le dispositif intermédiaire 5, une fois le dispositif intermédiaire 5 solidifié. Dans le cadre de cet exemple, la cuve 3 est placée pendant environ une heure (intervalle de temps T1) à une température de -80°C (conformément à l'invention, la cuve doit être placée à une température comprise entre - 5°C et -90°C pendant un premier intervalle de temps).

Pendant l'intervalle de temps T1, le dispositif intermédiaire 5 se solidifie : en effet, la température de l'enceinte 6 force le dispositif intermédiaire à geler, et la pression mécanique exercée par le gel du dispositif intermédiaire 5 assure la création d'un lien solide entre les molécules du matériau 10 qui va perdurer même après dégèle du dispositif.

Ainsi, à l'issue de l'intervalle de temps T1, la cuve 3 est retirée de l'enceinte réfrigérée 6 et la cuve est laissée à température ambiante (température de la pièce dans laquelle le procédé est mis en oeuvre, supérieure aux température de solidification du dispositif intermédiaire) pendant un second intervalle de temps T2 de sensiblement trois heures.

Le second intervalle de temps T2 est donc supérieur (au moins deux fois) au premier intervalle de temps T1.

Dans le cadre de ce mode de réalisation, l'étape de solidification est ainsi réalisée pendant un cycle comprenant le premier intervalle de temps T1 de solidification et le second intervalle de temps de mise à température ambiante.

Il est possible de réaliser cette étape de solidification en réalisant plusieurs cycles : après un premier cycle (T1 à une température de -80°C et T2 à température ambiante), il est possible de recommencer l'opération de solidification à T1 (-80°C) suivi de T2 (à température ambiante).

Le fait de réaliser plusieurs cycles permet de simuler le vieillissement du substitut tissulaire, pour qu'il correspond à l'âge de l'élément du corps dans lequel il est destiné à être implanté.

En effet, en réalisant plusieurs cycles successifs, on simule un état d'usage du dispositif. Ainsi, si le substitut tissulaire à créer est destiné à une personne ayant une vingtaine d'années, par exemple, un seul cycle sera nécessaire. On peut également envisager de réaliser quatre cycles successifs pour que l'état du substitut tissulaire 50 soit proche de l'état d'une artère d'un patient âgé, par exemple, de 60 ans.

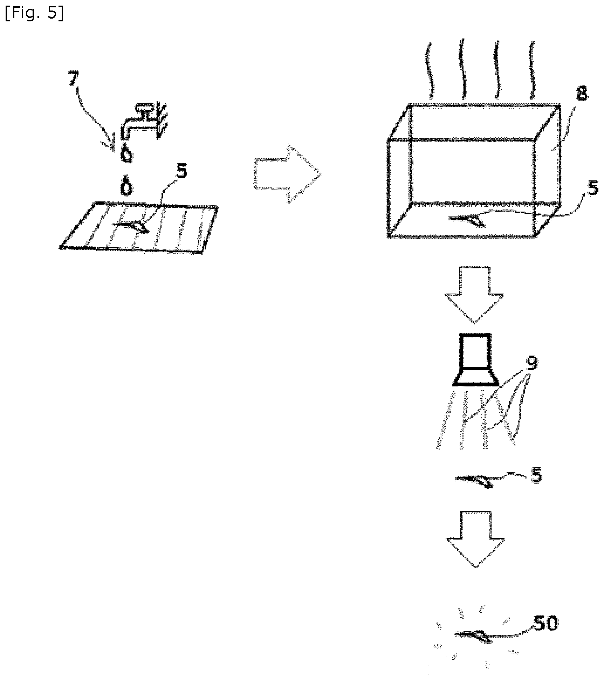

En effet, en réalisant plusieurs cycles successifs, on augmente le degré de cristallinité du dispositif tissulaire, ce qui a pour effet d'induire une rigidification et une perte des propriétés élastiques du substitut tissulaire. A l'issu du cycle assurant la solidification du dispositif intermédiaire 5, on procède au retrait du dispositif intermédiaire 5 de la cuve (en le saisissant manuellement ou avec un ustensile) et on procède à un rinçage 7 du dispositif intermédiaire 5 (voir figure 5).

Puis, il est prévu de sécher le dispositif 5.

Un séchage à l'air libre peut être envisagé.

Un séchage par lyophilisation est préférable à tout autre séchage pour maintenir au mieux la forme du dispositif obtenu : pour ce faire, le dispositif intermédiaire 5 est placé dans un caisson de lyophilisation 8 mettant en oeuvre une pompe à vide ; durant un temps de lyophilisation qui est tel que le dispositif intermédiaire 5 obtenu ne contient, tout au plus, que 3% d'eau.

Le dispositif obtenu se conserve alors mieux jusqu'à sa réhydratation avant implantation dans le corps humain.

Enfin, pour s'assurer qu'aucun agent contaminant ne se développe sur le dispositif, on prévoit de le décontaminer en projetant sur lui un rayonnement lumineux 9 décontaminant, du type Ultraviolets.

On obtient alors, à l'issue de toutes les étapes précitées, un substitut tissulaire 50 conforme à l'invention.

On comprend de la description qui précède comment l'invention permet de réaliser un substitut tissulaire 50 en biomatériau et imprimé en 3D, de façon sûr, simple et rapide.

On comprend également comment l'invention permet de réaliser des substituts tissulaires qui sont adaptés à l'âge des patients, car le procédé permet de réaliser des substituts tissulaires plus ou moins « usés » et correspondant à l'état des tissus du patient concerné.

## Revendications

1. Procédé de réalisation d'un substitut tissulaire (50) imprimé en 3D, mettant en oeuvre un dispositif d'impression 3D (2) comprenant une tête d'impression (21) équipée d'au moins une cartouche (20) de délivrance d'un matériau (10) à imprimer, ledit dispositif d'impression 3D (2) comportant une cuve (3) comprenant un fluide de contrainte (30), formant un gel, dans lequel ledit matériau (10) est imprimé, ladite tête d'impression (21) étant montée mobile en déplacement, ledit matériau (10) à imprimer se solidifiant sensiblement sous une première température de solidification et ledit fluide de contrainte (30) se solidifiant sensiblement sous une seconde température de solidification, ledit procédé étant **caractérisé**
**en ce que** ledit matériau (10) à imprimer délivré par la cartouche (20) comporte de l'alcool polyvinylique (40) et de la gélatine (41),
**en ce que** ladite seconde température de solidification du fluide de contrainte (30) est inférieure à ladite première température de solidification dudit matériau (10) à imprimer,
et **en ce que** ledit procédé comporte les étapes suivantes :
- solubilisation dudit matériau (10) à imprimer, de manière à obtenir un matériau (10) suffisamment fluide pour qu'il soit délivré par ladite cartouche (20),
- alimentation dudit dispositif d'impression 3D (2) avec ledit matériau (10) solubilisé,
- impression dudit matériau (10) dans ledit fluide de contrainte (30) dans la cuve (3), par déplacement de la tête d'impression (21) dans ladite cuve (3) et délivrance de ladite matière par ladite cartouche (20) de manière à former un dispositif intermédiaire (5),
- solidification dudit dispositif intermédiaire (5) en plaçant ladite cuve (3), pendant un premier intervalle de temps (T1), à une température inférieure à ladite première température de solidification, ladite température étant toutefois supérieure de ladite seconde température de solidification, puis en plaçant la cuve (3) à température ambiante pendant un second intervalle de temps (T2), ladite température ambiante étant supérieure auxdites première et seconde températures de solidification,
- retrait dudit dispositif intermédiaire (5) solidifié de ladite cuve (3),
- rinçage dudit dispositif intermédiaire (5) solidifié, et
- séchage dudit dispositif intermédiaire (5) pour obtenir ledit substitut tissulaire (50).

2. Procédé selon la revendication 1, **caractérisé en ce que** ledit matériau (10) à imprimer comporte sensiblement entre 10 et 25% en poids d'alcool polyvinylique (40) et sensiblement entre 0,5 et 2% en poids de gélatine (41), de préférence sensiblement 20% en poids d'alcool polyvinylique (40) et sensiblement 1% en poids de gélatine (41).

3. Procédé selon la revendication 1 ou 2, **caractérisé en ce que** le fluide de contrainte (30) comporte sensiblement au moins 80% en poids d'éthanol et sensiblement entre 2 et 5% en poids d'un polymère synthétique d'acide acrylique réticulé, de préférence sensiblement 3,5% en poids.

4. Procédé selon l'une quelconque des revendications précédentes, **caractérisé en ce que** ladite solubilisation dudit matériau (10) à imprimer est réalisée à une température comprise entre sensiblement 90°C et 100 °C.

5. Procédé selon l'une quelconque des revendications précédentes, **caractérisé en ce que** ladite solidification dudit dispositif intermédiaire (5) est réalisée à une température comprise sensiblement entre -5°C et -90°C, de préférence à sensiblement -80°C.

6. Procédé selon l'une quelconque des revendications précédentes, **caractérisé en ce que** ladite solidification est réalisée en plaçant ladite cuve (3) à ladite température de solidification dans une enceinte réfrigérée (6) pendant sensiblement 1 heure (T1), puis en laissant la cuve (3) à température ambiante pendant sensiblement 3 heures (T2).

7. Procédé selon l'une quelconque des revendications précédentes, **caractérisé en ce que** l'étape de solidification est répétée au moins deux fois.

8. Procédé selon l'une quelconque des revendications précédentes, **caractérisé en ce que** le séchage est réalisé par lyophilisation (8) du dispositif intermédiaire (5) durant un temps de lyophilisation tel que le substitut tissulaire (50) obtenu à l'issu dudit temps de lyophilisation comprenne moins de 3% d'eau en poids.

9. Procédé selon l'une quelconque des revendications précédentes, **caractérisé en ce qu'**il comporte une ultime étape de décontamination (9) dudit substitut tissulaire (50).

10. Procédé selon la revendication 9, **caractérisé en ce que** ladite étape de décontamination est réalisée en soumettant ledit substitut tissulaire (50) à un rayonnement décontaminant (9).

## Patentansprüche

1. Verfahren zur Herstellung eines 3D-gedruckten Gewebeersatzes (50) unter Verwendung einer 3D-Druckvorrichtung (2), die einen Druckkopf (21) umfasst, der mit mindestens einer Kartusche (20) zur Lieferung eines Druckmaterials (10) ausgestattet ist, wobei die 3D-Druckvorrichtung (2) einen Behälter (3) umfasst, der ein gelbildendes Begrenzungsfluid (30) enthält, in welchem das Material (10) gedruckt wird, wobei der Druckkopf (21) für eine Verlagerung bewegbar angeordnet ist, wobei das Druckmaterial (10) sich bei einer ersten Verfestigungstemperatur erheblich verfestigt und das Begrenzungsfluid (30) sich bei einer zweiten Verfestigungstemperatur erheblich verfestigt, wobei das Verfahren **dadurch gekennzeichnet ist,**
**dass** das von der Kartusche (20) gelieferte Druckmaterial (10) Polyvinylalkohol (40) und Gelatine (41) umfasst,
**dass** die zweite Verfestigungstemperatur des Begrenzungsfluids (30) niedriger als die erste Verfestigungstemperatur des Druckmaterials (10) ist,
und **dass** das Verfahren die folgenden Schritte umfasst:
- Solubilisierung des Druckmaterials (10), um ein Material (10) zu erhalten, das ausreichend flüssig ist, um von der Kartusche (20) geliefert zu werden,
- Versorgung der 3D-Druckvorrichtung (2) mit dem solubilisierten Material (10),
- Druck des Materials (10) in dem Begrenzungsfluid (30) in dem Behälter (3) durch Verlagerung des Druckkopfs (21) in dem Behälter (3) und Lieferung des Materials durch die Kartusche (20), um eine Zwischenanordnung (5) zu bilden,
- Verfestigung der Zwischenanordnung (5), indem der Behälter (3) in einer ersten Zeitspanne (T1) auf eine Temperatur unterhalb der ersten Verfestigungstemperatur gebracht wird, wobei diese Temperatur jedoch oberhalb der zweiten Verfestigungstemperatur liegt, und indem der Behälter (3) dann in einer zweiten Zeitspanne (T2) auf eine Umgebungstemperatur gebracht wird, wobei die Umgebungstemperatur oberhalb der ersten und der zweiten Verfestigungstemperaturen liegt,
- Entfernung der verfestigten Zwischenanordnung (5) aus dem Behälter (3),
- Spülung der verfestigten Zwischenanordnung (5), und
- Trocknung der Zwischenanordnung (5), um den Gewebeersatz (50) zu erhalten.

2. Verfahren nach Anspruch 1, **dadurch gekennzeichnet, dass** das Druckmaterial (10) etwa 10 bis 25 Gew.-% Polyvinylalkohol (40) und etwa 0,5 bis 2 Gew.-% Gelatine (41), vorzugsweise etwa 20 Gew.-% Polyvinylalkohol (40) und etwa 1 Gew.-% Gelatine (41), enthält.

3. Verfahren nach Anspruch 1 oder 2, **dadurch gekennzeichnet, dass** das Begrenzungsfluid (30) in etwa mindestens 80 Gew.-% Ethanol und in etwa zwischen 2 und 5 Gew.-% eines synthetischen Polymers aus vernetzter Acrylsäure, vorzugsweise in etwa 3,5 Gew.-%, enthält.

4. Verfahren nach einem der vorhergehenden Ansprüche, **dadurch gekennzeichnet, dass** die Solubilisierung des Druckmaterials (10) bei einer Temperatur etwa zwischen 90°C und 100°C durchgeführt wird.

5. Verfahren nach einem der vorhergehenden Ansprüche, **dadurch gekennzeichnet, dass** das Verfestigen der Zwischenanordnung (5) bei einer Temperatur von etwa zwischen -5°C und -90°C, vorzugsweise bei etwa -80°C, durchgeführt wird.

6. Verfahren nach einem der vorhergehenden Ansprüche, **dadurch gekennzeichnet, dass** die Verfestigung durchgeführt wird, indem der Behälter (3) bei der Verfestigungstemperatur in einer gekühlten Kammer (6) für etwa 1 Stunde (T1) angeordnet wird und der Behälter (3) dann für etwa 3 Stunden (T2) bei Raumtemperatur belassen wird.

7. Verfahren nach einem der vorhergehenden Ansprüche, **dadurch gekennzeichnet, dass** der Schritt der Verfestigung mindestens zweimal wiederholt wird.

8. Verfahren nach einem der vorhergehenden Ansprüche, **dadurch gekennzeichnet, dass** die Trocknung durch eine Lyophilisierung (8) der Zwischenanordnung (5) während einer Lyophilisierungszeit durchgeführt wird, derart, dass der nach der Lyophilisierungszeit erhaltene Gewebeersatz (50) weniger als 3 Gew.-% Wasser enthält.

9. Verfahren nach einem der vorhergehenden Ansprüche, **dadurch gekennzeichnet, dass** es einen abschließenden Schritt zur Dekontamination (9) des Gewebeersatzes (50) umfasst.

10. Verfahren nach Anspruch 9, **dadurch gekennzeichnet, dass** der Schritt zur Dekontamination durchgeführt wird, indem der Gewebeersatz (50) einer dekontaminierenden Strahlung (9) ausgesetzt wird.

## Claims

1. Method for producing a 3D-printed tissue substitute (50), utilizing a 3D printing device (2) comprising a print head (21) equipped with at least one printing material (10) delivery cartridge (20), said 3D printing device (2) including a tank (3) comprising a yield stress fluid (30), forming a gel in which said material (10) is printed, said print head (21) being mounted mobile in displacement, said printing material (10) solidifying substantially under a first solidification temperature and said yield stress fluid (30) solidifying substantially under a second solidification temperature, said method being **characterized**
**in that** said printing material (10) delivered by the cartridge (20) includes polyvinyl alcohol (40) and gelatin (41),
**in that** said second solidification temperature of the yield stress fluid (30) is lower than said first solidification temperature of said printing material (10),
and **in that** said method includes the following steps:
- solubilizing said printing material (10) so as to obtain a sufficiently fluid material (10) so that it is delivered by said cartridge (20),
- supplying said 3D printing device (2) with said solubilized material (10),
- printing said material (10) in said yield stress fluid (30) in the tank (3) by moving the print head (21) into said tank (3) and delivery of said material by said cartridge (20) so as to form an intermediate device (5),
- solidifying said intermediate device (5) by placing said tank (3) at a temperature lower than said first solidification temperature for a first time interval (T1), said temperature however being higher than said second solidification temperature, then by placing the tank (3) at ambient temperature for a second time interval (T2), said ambient temperature being higher than said first and second solidification temperatures,
- removing said solidified intermediate device (5) from said tank (3),
- rinsing said solidified intermediate device (5), and
- drying said intermediate device (5) in order to obtain said tissue substitute (50).

2. Method according to claim 1, **characterized in that** said printing material (10) includes substantially between 10 and 25% by weight polyvinyl alcohol (40) and substantially between 0.5 and 2% by weight gelatin (41), preferably substantially 20% by weight polyvinyl alcohol (40) and substantially 1% by weight gelatin (41).

3. Method according to claim 1 or 2, **characterized in that** the yield stress fluid (30) includes substantially at least 80% by weight ethanol and substantially between 2 and 5% by weight of a cross-linked acrylic acid synthetic polymer, preferably substantially 3.5% by weight.

4. Method according to any one of the preceding claims, **characterized in that** said solubilization of said printing material (10) is carried out at a temperature comprised between substantially 90°C and 100°C.

5. Method according to any one of the preceding claims, **characterized in that** said solidification of said intermediate device (5) is carried out at a temperature comprised substantially between -5°C and -90°C, preferably at substantially -80°C.

6. Method according to any one of the preceding claims, **characterized in that** said solidification is carried out by placing said tank (3) at said solidification temperature in a refrigerated cabinet (6) for substantially 1 hour (T1), then by leaving the tank (3) at ambient temperature for substantially 3 hours (T2).

7. Method according to any one of the preceding claims, **characterized in that** the solidification step is repeated at least twice.

8. Method according to any one of the preceding claims, **characterized in that** the drying is carried out by lyophilization (8) of the intermediate device (5) during a lyophilization time such that the tissue substitute (50) obtained at the end of said lyophilization time comprises less than 3% by weight water.

9. Process according to any one of the preceding claims, **characterized in that** it includes a final step of decontamination (9) of said tissue substitute (50).

10. Method according to claim 9, **characterized in that** said decontamination step is carried out by subjecting said tissue substitute (50) to decontaminating radiation (9).
